# EUROPEAN PATENT APPLICATION

(11) **EP 4 042 972 A1**
(43) Date of publication of application: **17.08.2022**
(21) Application number: 20874388.0
(22) Date of filing: 28.09.2020
(51) Int. Cl.: A61F 2/24

(54) **IMPROVED LEAFLET OF CARDIAC VALVE, AND CARDIAC VALVE PREFORM, CARDIAC VALVE, AND PROCESSING METHOD THEREFOR**

(30) Priority: 08.10.2019 CN 201910949679
(71) Applicant: Venus MedTech (HangZhou), Inc., Hangzhou, Zhejiang 310052 (CN)
(72) Inventor: QI, Jesse Jun, Hangzhou, Zhejiang 310052 (CN); ZENG, Min Frank, Hangzhou, Zhejiang 310052 (CN); ZI, Zhenjun, Hangzhou, Zhejiang 310052 (CN)
(74) Representative: KIPA AB
(86) International application number: PCT/CN2020/118540
(87) International publication number: WO 2021/068788

(57) **Abstract**

Disclosed are an improved leaflet of a cardiac valve, and a cardiac valve preform, a cardiac valve, and a processing method therefor. The improved leaflet of a cardiac valve comprises a leaflet body (1), wherein the leaflet body (1), in a height direction, is provided with a movable edge (12) on the top side and a fixed edge (11) on the bottom side, and the movable edge (12) and the fixed edge (11) extend in the width direction of the leaflet body (1) and are connected to connecting lugs (10) on respective sides. Each of the connecting lugs (10) comprises a lifting part (2), the lifting part (2) extending in the height direction and crossing the movable edge (12), wherein the movable edge (12) extends and intersects with the inner side of the lifting part (2), and the fixed edge (11) extends and intersects with the outer side of the lifting part (2); and a first wing (3), wherein the first wing (3) is connected to the inner side of the lifting part (2), a first fold line (L1) is provided between the first wing (3) and the inner side of the lifting part (2). The side of the first wing (3) that faces the movable edge (12) is a bottom edge (31), and a stress release opening (32) allowing the first wing (3) to be folded along the first fold line (L1) is reserved between the bottom edge (31) and the movable edge (12).

## Description

### TECHNICAL FIELD

The present invention relates to the technical field of medical devices, and more particularly to improved leaflets for heart valves and heart valve preforms, heart valves, and preparing methods.

### BACKGROUND

A known leaflet for the heart valve generally includes a leaflet body, which has a three-dimensional configuration in use. In a flattened state before being sewn, the leaflet body has a movable edge on the top side and a fixed edge on the bottom side in the height direction. The fixed edge is configured to be fixed with the support (typically a stent or with an inner covering film). The movable edges of the leaflets cooperate to open or close the blood flow passage. The movable edge and the fixed edge extend in the width direction of the leaflet body and meet to the connecting ears on the respective sides. The connecting ears are fixed to the stent and primarily function to pull the movable edges, and keep flexible opening and closing of the movable edges thereby ensuring the sealing effect.

In the prior art, the connection ear is directly sewn and fixed to the support, or slightly folded to form a reinforced portion and then sewn and fixed to the support. It is difficult to ensure the connection strength by directly sewing to the support. In the case where the connection ear is folded, due to the shape of the connecting ear and the positional relationship between the connecting ear and the leaflet body, the sealing performance and the stress condition of the movable edges, especially at the side close to the support, are not ideal. In addition, the folded portions are not turned over in flat, which is likely to cause a gap, with a risk of thrombus.

### SUMMARY

The present application provides an improved leaflet for a heart valve in order to optimize the stress of the movable edge of the leaflet body and to reduce the risk of thrombus in the folded portion.

The improved leaflet for the heart valve according to the present application comprises a leaflet body which, in a height direction, has a movable edge on a top side and a fixed edge on a bottom side, the movable edge and the fixed edge extending in a width direction of the leaflet body and connected to connecting ears on respective sides, wherein the connecting ear comprises:

A pulling portion extending in the height direction and crossing over the movable edge, the movable edge extending to and meeting with an inner side of the pulling portion, and the fixed edge extending to and meeting with an outer side of the pulling portion; and
A first flap connected to the inner side of the pulling portion, with a first fold line defined between the first flap and the inner side of the pulling portion, wherein one side of the first flap facing the movable edge is a bottom edge, and a stress release opening is defined between the bottom edge and the movable edge to allow the first flap to be turned over along the first fold line.

In the following, a number of alternatives are provided, but not as additional limitations to the above-mentioned solution, but merely as further additions or preferences. Without technical or logical contradiction, the alternatives can be combined with the above-mentioned solution independently or in combination.

Optionally, a middle portion of the fixed edge protrudes toward the bottom side of the leaflet body in a parabolic shape; and the movable edge extends straightly, or a middle portion of the movable edge also protrudes toward the bottom side of the leaflet body.

Optionally, a line connecting a midpoint of the movable edge and a midpoint of the fixed edge is a center line of the leaflet body, and the connecting ears on two sides of the leaflet body are symmetrically or asymmetrically distributed on two sides of the center line.

Optionally, the pulling portion is a strip, and in a length direction of the strip, one side of the pulling portion is a bottom edge which is a part of the fixed edge, and the other side of the pulling portion is a top edge, and wherein a top edge of the first flap and the top edge of the pulling portion are flush with or offset from each other.

Optionally, portions of the top edges of the first flap and the pulling portion adjacent to the first fold line are symmetrical about the first fold line.

Optionally, one end of the top edge of the first flap is flush with the top edge of the pulling portion, and the other end extends in the width direction of the leaflet body or is inclined toward the bottom edge of the first flap.

Optionally, the pulling portion extends with the same width after crossing over an end of the movable edge.

Optionally, the first flap is a strip and extends with the same width after crossing over an end of the movable edge.

Optionally, one end of the bottom edge of the first flap meets the inner side of the pulling portion, and the other end extends in the width direction of the leaflet body or obliquely with respect to the width direction.

Optionally, a width of the first flap is less than a width of the pulling portion and greater than 50% of the width of the pulling portion.

Optionally, the first flap is configured to be overlapped with the pulling portion in use.

Optionally, the first flap has a corner at a bottom thereof and at a side thereof away from the pulling portion, and in the height direction of the leaflet body, the corner is located below an end of the movable edge.

Optionally, the stress release opening is strip-shaped and extends with the same width or is narrowed gradually.

Optionally, the stress release opening is strip-shaped and extends along a straight line or a curved line.

Optionally, the stress release opening has a closed end adjacent to the first fold line, and the closed end has an arcuate inner edge.

Optionally, the stress release opening extends along a curved line and curves gradually toward a top edge of the first flap as the stress release opening approaches the first fold line.

Optionally, the outer side of the pulling portion is further connected with a second flap, a bottom edge of the second flap is connected with an end of the fixed edge, and the connection portion is a turning point, and wherein a second fold line extends from the turning point upward between the pulling portion and the second flap.

Optionally, the second flap is a strip, and a middle portion of the second flap in a length direction thereof extends with the same width in the height direction of the leaflet body.

Optionally, a width of the second flap is less than a width of the pulling portion and greater than 50% of the width of the pulling portion.

Optionally, the second flap is configured to be overlapped with the pulling portion in use.

Optionally, the second flap and the first flap are configured to be respectively overlapped on two sides of the pulling portion or on the same side of the pulling portion in use.

Optionally, the second flap and the first flap are configured to be respectively overlapped on the same side of the pulling portion in use, with the first flap sandwiched between the second flap and the pulling portion.

Optionally, an angle at the turning point is an obtuse angle.

Optionally, one end of a top edge of the second flap is flush with a top edge of the pulling portion, and the other end extends in the width direction of the leaflet body or is inclined toward the bottom edge of the second flap.

Optionally, one end of the bottom edge of the second flap is flush with a bottom edge of the pulling portion, and the other end extends in the width direction of the leaflet body or is inclined toward a top edge of the second leaflet.

Optionally, an outer side of the second flap is further connected with a third flap with a third fold line defined between the second flap and the third flap, and the third flap is configured to be directly or indirectly attached to a support in use.

Optionally, top and bottom edges of the third flap are gradually inclined toward each other while extending outward.

Optionally, a width of the third flap is greater than a width of the second flap.

Optionally, the fixed edge has a larger span than the movable edge in the width direction of the leaflet body.

The present application further provides a heart valve preform comprising at least two leaflets and an inner covering film, wherein the leaflet is the improved leaflet for the heart valve according to the present application, and wherein the pulling portion and the first flap of the same leaflet are overlapped with each other to form a reinforced portion, and the reinforced portions of two adjacent leaflets are attached to each other to form an assembly of the reinforced portions; and the inner covering film is partially folded to wrap the assembly of the reinforced portions, and the wrapping portions are fixed by threading suture; and the fixed edges of the leaflets are fixed with the inner covering film.

The present application further provides a heart valve comprising a stent having a blood flow passage therein, in which three leaflets for controlling opening and closing of the blood flow passage are arranged, and wherein the leaflet is the improved leaflet for the heart valve according to the present application, and the movable edges of the leaflets cooperate with each other and have a closed state of being engaged with each other and an open state of being separated from each other.

Optionally, an inner covering film is provided on an inner wall of the stent, and the fixed edges and the pulling portions of the leaflets are all connected with the inner covering film. Optionally, the inner covering film is fixed with the inner wall of the stent by a first suture, and the pulling portion and the first flap are overlapped to form a reinforced portion, and the reinforced portions of two adjacent leaflets are attached to each other to form an assembly of the reinforced portions; and the inner covering film is partially folded to wrap the assembly of the reinforced portions, and the wrapping portions are fixed by a second suture.

Optionally, an outer side of the pulling portion is further connected with a second flap, and the reinforced portion further comprises the second flap overlapping with the pulling portion.

Optionally, the outer side of the second flap is further connected with a third flap extending in a circumferential direction of the stent and contacting the inner covering film, with the contact portion connected with the stent by a third suture.

Optionally, the stent has a structure of cells with each cell having edge strips and a hollowed-out area surrounded by the edge strips, and the third flaps of two adjacent leaflets are fixed with the edge strips of one of the cells by the third suture.

Optionally, at least a section of the stent in an axial direction is a straight cylindrical section, and the pulling portion is axially located on the straight cylindrical section, and an extension direction of the pulling portion is parallel to an axial direction of the straight cylindrical section.

Optionally, the heart valve is a pulmonary valve.

The present application further provides a method for preparing a heart valve, comprising connecting leaflets for the heart valve with an inner covering film to prepare a preform, and then fixing the preform to a stent, wherein the leaflet is the improved leaflet for the heart valve according to the present application, and preparing the preform comprises:
Overlapping the pulling portion with the first flap of the same leaflet to form a reinforced portion;
Attaching the reinforced portions of two adjacent leaflets together to form an assembly of the reinforced portions;
Folding the inner covering film partially to wrap the assembly of the reinforced portions, and threading suture through and thus fixing the wrapping portions; and
Fixing the fixed edges of the leaflets to the inner covering film.

In the present application, by improving the shape of the connecting ear and the positional relationship between the connecting ear and the leaflet body, the force applied to the movable edge is reasonably distributed, and the folded portions are as flat as possible thereby eliminating unnecessary stress and reducing the risk of thrombus.

The heart valve according to the present application can be used not only as an interventional instrument, but also as a surgical instrument, which is applicable to many scenes. Further, the service life of the heart valve is further improved by a reasonable arrangement of the portions of the connecting ear and the fold lines.

### BRIEF DESCRIPTION OF THE DRAWINGS

FIG. 1 is a schematic view of a leaflet for a heart valve of one embodiment according to the present application;
FIG. 2 is a schematic view of the leaflet for the heart valve shown in FIG. 1, with a first flap being turned over;
FIG. 3 is a schematic view of a leaflet for a heart valve of another embodiment according to the present application;
FIG. 4 is a schematic view of the leaflet for the heart valve shown in FIG. 3, with a first flap being turned over;
FIG. 5 is a schematic view of the leaflet for the heart valve shown in FIG. 3, with a second flap being turned over;
FIG. 6 is a schematic view of the leaflet for the heart valve shown in FIG. 5, with thrid flaps being turned over;
FIG. 7 is a schematic view of folding the connecting ear of the leaflet for the heart valve according to the present application;
FIG. 8 is a schematic view of a partial connection between the connecting ear of the leaflet for the heart valve according to the present application and the inner covering film;
FIG. 9 is a schematic view shown the connection of the leaflets for the heart valve according to the present application, the inner covering film and the stent;
FIG. 10 is a schematic view of the leaflets for the heart valve shown in FIG. 9 in an open state; and
FIG. 11 is a schematic view of a pulmonary valve according to the present application.

The reference numerals are listed below:
L0, center line; L1, first fold line; L2, second fold line; L3, third fold line; A, end of the movable edge; B, end of the fixed edge; C, sewing area; D, sewing area; H1, height direction; H2, height direction; W0, width direction; W1, width direction; W2, width direction; a, first corner; β1, first inclination angle; β2, second inclination angle; β3, third inclination angle;
1, leaflet body; 10, connecting ear; 11, fixed edge; 12, movable edge; 12A, movable edge; 12B, movable edge;
2, pulling portion, 21, bottom edge, 22, top edge;
3. first flap; 31, bottom edge; 32, stress release opening; 33, corner; 34, top edge;
4, second flap; 41, bottom edge; 42, top edge;
5, third flap; 51, bottom edge; 52, top edge;
6, inner covering film;
7, stent.

### DESCRIPTION OF THE EMBODIMENTS

The technical solutions according to the embodiments of the present disclosure will be described in combination with the drawings according to the embodiments of the present disclosure. The described embodiments represent some but not all the possible embodiments.

It should be noted that, when a component is "connected" with another component, it may be directly connected to another component or may be indirectly connected to another component through a further component. When a component is "provided" on another component, it may be directly provided on another component or may be provided on another component through a further component.

Unless otherwise defined, all technical and scientific terms used herein have the same meaning as commonly understood by a person skilled in the art. The terms in the description of the present disclosure are used to describe specific embodiments, and not to limit the present disclosure. The term "and/or" used herein includes any combinations of one or more of the listed options, as well as the combination of all of the listed options.

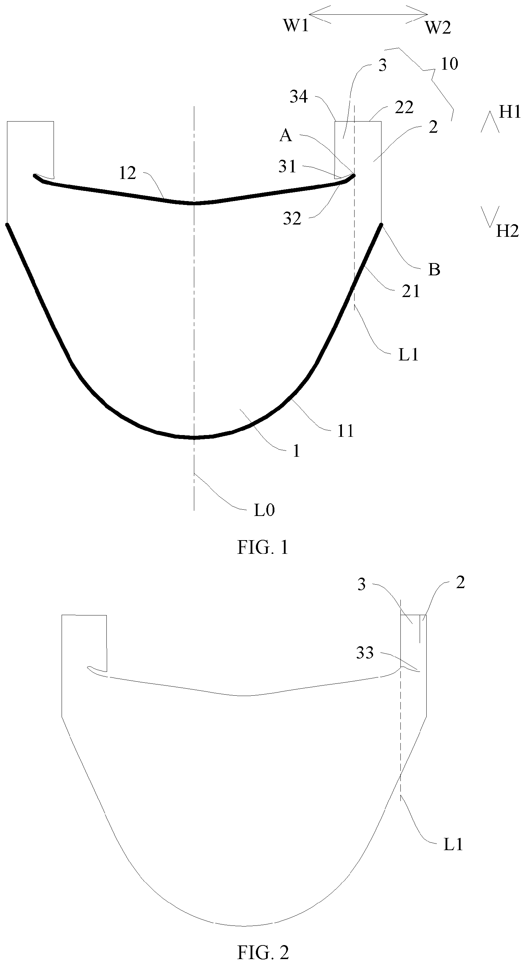

Referring to FIGS. 1 and 2, in one embodiment according to the present application, an improved leaflet for a heart valve is provided. The improved leaflet includes a leaflet body 1. The leaflet body 1 has, in the height direction, a movable edge 12 on the top side and a fixed edge 11 on the bottom side. The movable edge 12 and the fixed edge 11 respectively extend in the width direction of the leaflet body 1 and are connected to the respective connecting ears 10. The movable edge 12 and the fixed edge 11 are shown in FIG. 1 by respective bold solid lines.

The connecting ear 10 includes:
A pulling portion 2, which extends in the height direction and crosses over the movable edge 12; the movable edge 12 extends to and connects with the inner side of the pulling portion, and the fixed edge 11 extends to and connects with the outer side of the pulling portion; and
A first flap 3, which is connected with the inner side of the pulling portion, with a first fold line L1 defined between the first flap 3 and the inner side of the pulling portion; one side of the first flap 3 towards the movable edge 12 is defined as a bottom edge 31, and a stress release opening 32 is defined between the bottom edge 31 and the movable edge 12 to allow the first flap 3 to be turned over along the first fold line L1.

The leaflet for the heart valve has a three-dimensional configuration in space in use. Unless otherwise specified, in this application, the structure and shape of the leaflet for the heart valve are described referring to the flattened state. For example, as shown in FIG. 1, the lateral direction (left-right direction) shown in FIG. 1 is the width direction of the leaflet body 1. The leaflet body 1 has an approximate center line L0, although it is not strictly required to be symmetrical in structure. Two connecting ears are respectively distributed at two sides of the center line L0. The side of the right connecting ear 10 facing the central line L0 is the inner side. For example, the width direction W1 shown in FIG. 1 directs toward the inner side, while the width direction W2 directs toward the outer side. The same applies for the left connecting ear in FIG. 1.

The extension direction of the center line L0 is the height direction of the leaflet body 1. As shown in the figure, the height direction H1 can be regarded as pointing toward the upper or top side, and the height direction H2 can be regarded as pointing toward the lower or bottom side. It should be noted that upper and lower sides, and left and right are relative concepts, which are referred to the orientation of FIG. 1 only for ease of description.

The leaflet body 1 and the connecting ears are formed in one piece. The material can use the known biocompatible materials and should have necessary mechanical properties, which is not the focus of the present application and the prior art can be referred therefor.

The fixed edge 11 is configured to be connected to a support (hereinafter, a stent is taken as an example). A blood flow passage is defined in the interior of the support. The movable edge 12 cooperates with the adjacent other leaflet body(bodies) for controlling the opening or closing of the blood flow passage.

The heart valve in this embodiment can be used as an interventional instrument, that is, the heart valve in this embodiment can be implanted in the human body in an interventional manner. Alternatively, the heart valve in this embodiment can be used as a surgical instrument, that is, the heart valve in this embodiment can be implanted in the human body in a conventional surgical manner. Implanting the heart valve in which manner does not substantially affect the structure of the heart valve.

In this embodiment, the pulling portion 2 is configured as a strip with a certain width, which extends along the height direction H1 and crosses over the movable edge 12. That is, the top side of the pulling portion 2 obviously extends out of the movable edge 12. The end of the movable edge A meets the inner side of the pulling portion, i.e., the side of the pulling portion 2 facing the center line L0. The end of the fixed edge B meets the outer side of the pulling portion 2, that is, the side of the pulling portion 2 facing away from the center line L0. Since the end of the movable edge A and the end of the fixed edge B meet the inner and outer sides of the pulling portion 2, respectively, the distance between the end of the movable edge A and the end of the fixed edge B in the width direction is the width of the pulling portion 2.

The first flap 3, in use, is configured to be turned over along the first fold line L1 to overlap on the pulling portion 2 to form a reinforced portion, which is configured to be connected to the stent (or an inner covering film on the stent) by sewing. As the extension of the pulling portion 2 according to the present application is particularly improved and defined, the resulted reinforced portion ensures the connection with the stent in use, and more importantly, it provides a pulling force to the leaflet body 1 in the height direction, thereby pulling the movable edge 12 and ensuring the flexible opening and closing of the movable edge 12.

Depending on the anatomy of the pulmonary artery, one section of the stent is approximately a straight cylindrical section, and the extension of the pulling portion 2 relative to the leaflet body 1 makes it particularly suitable to be sewn on the straight cylindrical section to further improve the pulling effect.

In order to ensure the smooth turning over of the first flap 3 and to avoid unnecessary stress, the stress release opening 32 is defined between the bottom edge 31 of the first flap 3 and the movable edge 12. The stress release opening 32 can be formed by cutting, which can prevent the moving first flap 3 from pulling the movable edge 12, in such a way that the movable edge 12 can be more flat, and when the leaflets of two heart valves are close to each other, the movable edges 12 of the two heart valves can better contact with each other without folds, thereby ensuring the sealing effect.

Depending on the material of the leaflet, the stress release opening 32 can be configured as a slit by cutting, that is, the slit has no width when closed. Alternatively, the stress release opening 32 can be formed by locally cutting, that is, the stress release opening 32 has a width relative to the slit, the width can vary with the extension of the stress release opening 32 or not. The end of the stress release opening 32, i.e., the closed portion thereof, prefers to take an arc transition to reduce the risk of being torn.

In the case where the leaflet is woven, the stress release opening 32 can be reserved when weaving, and a locking stitch suture is preferred.

The fixed edge 11 is generally parabolic, and as shown in FIG. 1, the middle portion of the fixed edge 11 (i.e., the portion of the fixed edge 11 near the center line L0) protrudes toward the bottom side of the leaflet body 1.

The movable edge 12 in the closed state can extend straightly or the middle portion thereof can protrude toward the bottom side of the leaflet body. As shown in FIG. 1, the middle portion of the movable edge 12 protrudes slightly.

In most cases, the leaflet body 1 has a symmetrical structure. In one embodiment, the line connecting the midpoints of the movable edge 12 and the fixed edge 11 is the center line L0 of the leaflet body 1. The connecting ears of the leaflet body 1 on two sides are symmetrically arranged on two sides of the center line L0. In other embodiments and in the case where the shapes of the connecting ears on two sides are slightly different, the connecting ears on two sides of the leaflet body 1 are arranged asymmetrically on two sides of the center line L0.

The pulling portion 2 and the first flap 3 have top edges at the side in the height direction H1, that is, the side in which the movable edge 12 faces away from the fixed edge 11, and bottom edges at the side in the height direction H2.

In one embodiment, the pulling portion 2 is configured as a strip. In the length direction of the strip, one side of the pulling portion 2 is the bottom edge 21 which forms as a part of the fixed edge 11, and the other side of the pulling portion 2 is the top edge 22 which is flush with or offset from the top edge 34 of the first flap 3.

Since the fixed edge 11 intersects the outer side of the pulling portion 2, the end region of the fixed edge 11 can also be considered as the bottom edge 21 of the pulling portion 2. The bottom edge 21 extends smoothly over the first fold line L1, without a visible turning point.

In the case where the top edge 34 of the first flap 3 and the top edge 22 of the pulling portion 2 are flush with each other, that is, the outer side of the top edge of the first flap 3 and the inner side of the top edge of the pulling portion 2 are equal in height, as shown in FIG. 1 according to the present embodiment, the top edge 34 of the first flap 3 and the top edge 22 of the pulling portion 2 are at the same height and extend along the width direction. Alternatively, the top edge 34 of the first flap 3 and the top edge 22 of the pulling portion 2 can extend obliquely, and the extension path can be in a straight line or a curved line. Preferably, the top edge 34 of the turned first flap 3 coincides with the top edge 22 of the pulling portion 2, that is, the portions of the two adjacent to the first fold line L1 are symmetrical about the first fold line L1.

In other embodiments, the outer side of the top edge of the first flap 3 is lower than the inner side of the top edge of the pulling portion 2, i.e. the two are offset from each other.

In other embodiments, one end of the top edge 34 of the first flap 3 is flush with the top edge 22 of the pulling portion 2, and the other end extends in the width direction of the leaflet body 1 or is inclined toward the bottom edge 34 of the first flap 2.

In the case where the top edge 34 of the first flap 3 is inclined toward the bottom edge 34 of the first flap 2, the lower the top edge 34 of the first flap 3 is, the top edge 34 of the first flap 3 is closer to the center line L0. A proper inclination for the top edge 34 of the first flap 3 can avoid a protruding corner after overlapping on the pulling portion 2.

In the case where the pulling portion 2 is configured as a strip, in the length direction thereof, the pulling portion 2 preferably extends in the same width after extending over the movable edge 12. As shown in FIG. 1, the first fold line L1 is parallel to the outer side of the pulling portion 2, and the portion of the pulling portion 2 above the end of the movable edge A extends with the same width. Similarly, it is preferable that the first flap 3 is configured as a strip, and in the length direction thereof, the portion of the first flap 3 above the end of the movable edge A also extends with the same width so that the first fold line L1 is also parallel to the inner side of the first flap 3.

The bottom edge 31 of the first flap 3 is spaced apart from the movable edge 12, and the stress release opening 32 is provided therebetween. In one embodiment, one end of the bottom edge 31 of the first flap 3 meets the inner side of the pulling portion 2, and the other end extends in the width direction of the leaflet body 1 or obliquely with respect to the width direction.

Referring to FIG. 1, the bottom edge 31 of the first flap 3 shown in FIG. 1 does not extend in the width direction of the leaflet body 1. The bottom edge 31 can extend in a straight line or a curved line. As shown in the figure, the lower the bottom edge 31 is, the bottom edge 31 is closer to the center line L0.

Since the pulling portion 2 and the first flap 3 respectively extend with the same width after crossing over the movable edge 12, in a preferred embodiment, the width of the first flap 3 is less than the width of the pulling portion 2 and greater than 50% of the width of the pulling portion 2.

The width of the first flap 3 is smaller than the width of the pulling portion 2 so that the first flap 3 does not exceed the outer side of the pulling portion 2 after the two are overlapped with each other. Further, the width of the first flap 3 is greater than 50% of the width of the pulling portion 2 so that the first flap 3 covers more than half of the width of the pulling portion 2 after the pulling portion 2 and the first flap 3 are overlapped with each other, thereby facilitating the sewing to ensure the necessary strength.

In use, the first flap 3 and the pulling portion 2 are overlapped with each other, and the suture after the two are overlapped with each other extends substantially along the height direction and is located at the middle of the pulling portion 2 in the width direction.

As the movable edge is configured to move constantly in use, the connection strength between the end of the movable edge A and the stent should be high. In a preferred embodiment, a corner 33 is provided at the bottom of the first flap and at the side away from the pulling portion 2, which is located below the end of the movable edge A in the height direction of the leaflet body 1. In use, after the first flap 3 and the pulling portion 2 are overlapped with each other, the first flap 3 can be further sewn at the corner 33 to improve the local connection strength. Since the corner 33 is located below the end of the movable edge A, the opening and closing of the movable edge 12 are not affected.

The stress release opening 32 is provided so that when the first flap 3 is turned over, the first flap 3 is prevented from pulling the movable edge 12 and the movable edges 12 can be closed flatly thereby ensuring the sealing effect.

In one embodiment, the stress release opening 32 is strip-shaped, and extends with the same width or is narrowed gradually. The strip-shaped stress release opening 32 can be simply provided by cutting, and the extension path of the strip can be in the form of a straight line or a curved line. The end of the stress release opening 32 extends to the first fold line L1 and is closed. In order to relieve the stress at the end of the stress release opening 32, in a preferred embodiment, the closed end of the stress release opening 32 is adjacent to the first fold line L1, and the inner edge of the closed end is arcuate.

In one embodiment, referring to FIG. 1, the stress release opening 32 extends along a curved line and curves gradually toward the top edge 34 of the first flap as it approaches the first fold line L1. That is, the stress release opening 32 is upturned away from the center line L0, with the corner 33 formed at the bottom of the first flap 3.

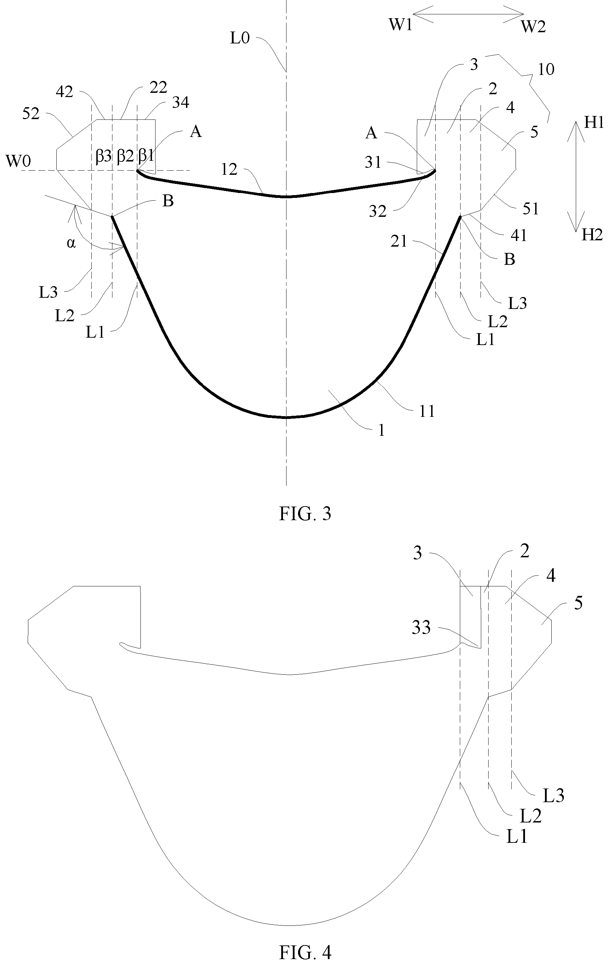

Referring to FIGS. 3 to 6, in another embodiment and in addition to the above embodiments, a second flap 4 is further provided which is connected with the outer side of the pulling portion 2, and the bottom edge of the second flap 4 is connected with an end of the fixed edge 11, wherein the connection portion is a turning point. A second fold line L2 extends upward from the turning point between the pulling portion 2 and the second flap 4.

In this embodiment, an improved leaflet for a heart valve is provided. The improved leaflet includes a leaflet body 1. The leaflet body 1 has, in the height direction, a movable edge 12 on the top side and a fixed edge 11 on the bottom side. The movable edge 12 and the fixed edge 11 respectively extend in the width direction of the leaflet body 1 and are connected to the respective connecting ears 10. The movable edge 12 and the fixed edge 11 are shown in FIG. 3 by respective bold solid lines.

The connecting ear 10 includes:
A pulling portion 2, which extends in the height direction and crosses over the movable edge 12; the movable edge 12 extends to and connects with the inner side of the pulling portion, and the fixed edge 11 extends to and connects with the outer side of the pulling portion;
A first flap 3, which is connected with the inner side of the pulling portion, with a first fold line L1 defined between the first flap 3 and the inner side of the pulling portion; one side of the first flap 3 towards the movable edge 12 is defined as a bottom edge 31, and a stress release opening 32 is defined between the bottom edge 31 and the movable edge 12 to allow the first flap 3 to be turned over along the first fold line L1; and
A second flap 4, which is connected with the outer side of the pulling portion, and the bottom edge of the second flap 4 is connected with an end of the fixed edge 11, wherein the connection portion is a turning point. A second fold line L2 extends upward from the turning point between the pulling portion 2 and the second flap 4.

In this embodiment, the description about the height, width, inner side, outer side and the like can refer to the foregoing and would not be repeated here anymore. In addition, in order to avoid influencing the view of the figure, the top sides, the bottom sides and the like of the connecting ears 10 arranged on two sides of the center line L0 can refer to the orientation shown in FIG. 3.

In the present embodiment, the second flap 4 is configured to be overlapped with the first flap 3 and the pulling portion 2 in use to further improve the fatigue resistance of the resulted reinforced portion and ensure the connection strength with the stent.

The first flap 3 and the second flap 4 are respectively located at the inner and outer sides of the pulling portion 2. The order of overlapping the first flap 3 and the second flap 4 is not strictly limited. In assemble with the adjacent leaflet(s) for the heart valve, the first flap 3, the second flap 4 and the pulling portion 2 of the same leaflet for the heart valve can be overlapped with each other in advance. Alternatively, the first flap 3, the second flap 4 and the pulling portion 2 of the same leaflet for the heart valve can be wound with the adjacent leaflet(s) for the heart valve and then, as a whole, be overlapped with each other.

The area covered by the second flap 4 after being turned over is preferably within the pulling portion 2. In one embodiment, the second flap 4 is strip-shaped and the middle portion thereof in its length direction extends with the same width in the height direction of the leaflet body 1. In other word, the second flap 4 has the same width at the middle portion thereof in its length direction, and can be appropriately narrowed near the top edge or the bottom edge thereof.

In one embodiment, the width of the second flap 4 is smaller than the width of the pulling portion 2 and greater than 50% of the width of the pulling portion 2. The width of the second flap 4 here refers to the width of the middle portion of the second flap 4, which is usually the widest portion thereof. The width of the second flap 4 is smaller than the width of the pulling portion 2 so that after the second flap 4 and the pulling portion 2 are overlapped with each other, the second flap 4 does not extend beyond the inner side of the pulling portion 2. Furthermore, the width of the second flap 4 is greater than 50% of the width of the pulling portion 2 so that the second flap 4 can cover more than half of the pulling portion 2 in the width direction after the second flap 4 and the pulling portion 2 are overlapped with each other, thereby facilitating the sewing to ensure the necessary strength.

In use, the second flap 4 and the pulling portion 2 are overlapped with each other. Regarding the specific overlapping order, the second flap 4 and the first flap 3 can be overlapped respectively on two sides of the pulling portion 2 or on the same side of the pulling portion 2. In a preferred embodiment, the second flap 4 and the first flap 3 are overlapped on the same side of the pulling portion 2, and the first flap 3 is sandwiched between the second flap 4 and the pulling portion 2. That is, the second flap 4 and the pulling portion 2 respectively contact two sides of the first flap 3 in the thickness direction.

The bottom edge 41 of the second flap 4 is connected with the end of the fixed edge B, and the connection portion is defined as a turning point. That is, the edge has a visible turning point, which is designed to avoid a blind angle at the second fold line L2 after the second flap 4 is turned over, where thrombus is likely to be formed. In a preferred embodiment, the angle at the turning point, i.e., the first corner α, is an obtuse angle. The first corner α is greater than 90 degrees, for example, between 105 and 150 degrees, preferably between 120 and 135 degrees. If the first corner α is too large, the turning point is not visible, and if the first corner α is too small, a sharp corner would be formed at the bottom edge 41 after the second flap 4 is turned over.

The shape of the top edge or bottom edge and the extension path of the second flap 4 can be understood referring to the width direction W0 of the leaflet body 1. In one embodiment, one end of the top edge 42 of the second flap 4 is connected with the top edge 22 of the pulling portion 2, and the other end extends in the width direction of the leaflet body 1 or is inclined toward the bottom edge 41 of the second flap 4. Inclining toward the bottom edge 41 of the second flap 4 means that the further away from the center line L0, the more the top edge 42 of the second flap 4 extends downward. The specific extension path of the top edge 42 of the inclined second flap 4 can be a straight line, a broken line or a curved line.

In one embodiment, one end of the bottom edge 41 of the second flap 4 is connected with the bottom edge 21 of the pulling portion 2, and the other end extends in the width direction of the leaflet body 1 or is inclined toward the top edge 42 of the second flap 4. Inclining toward the top edge 42 of the second flap 4 means that the further away from the center line L0, the more the bottom edge 41 of the second flap 4 extends upward. The specific extension path of the bottom edge 41 of the inclined second flap 4 can be a straight line, a broken line or a curved line.

Referring to FIGS. 3 to 6, in another embodiment and in addition to the above embodiments, an improved leaflet for a heart valve is provided. The improved leaflet includes a leaflet body 1. The leaflet body 1 has, in the height direction, a movable edge 12 on the top side and a fixed edge 11 on the bottom side. The movable edge 12 and the fixed edge 11 respectively extend in the width direction of the leaflet body 1 and are connected to the respective connecting ears 10. The connecting ear 10 includes:
A pulling portion 2, which extends in the height direction and crosses over the movable edge 12; the movable edge 12 extends to and connects with the inner side of the pulling portion, and the fixed edge 11 extends to and connects with the outer side of the pulling portion;
A first flap 3, which is connected with the inner side of the pulling portion, with a first fold line L1 defined between the first flap 3 and the inner side of the pulling portion; one side of the first flap 3 towards the movable edge 12 is defined as a bottom edge 31, and a stress release opening 32 is defined between the bottom edge 31 and the movable edge 12 to allow the first flap 3 to be turned over along the first fold line L1;
A second flap 4, which is connected with the outer side of the pulling portion, and the bottom edge of the second flap 4 is connected with an end of the fixed edge 11, wherein the connection is a turning point. A second fold line L2 extends upward from the turning point between the pulling portion 2 and the second flap 4; and
A third flap 5, which is connected with the outer side of the second flap 4, and a third fold line L3 is defined between the second flap 4 and the third flap 5. The third flap 5 is configured to directly or indirectly contact the support in use.

In use, the third flap 5 is not configured to be directly sewn to the second flap 4, but connected to the stent, so that the third flap 5 can shield the sewn portion of the second flap 4 and the pulling portion 2, and can be sewn and fixed to the stent to further increase the connection strength, furthermore, it can drive the reinforced portion formed by the first flap 3, the second flap 4 and the pulling portion 2 to be closer to the inner wall of the stent, thereby reducing the interference on the blood flow passage.

The bottom edge 51 and the top edge 52 of the third flap 5 are closer to each other as the third flap 5 extends outward from the third fold line L3, that is, the top edge 52 and the bottom edge 51 of the third flap 5 are gradually inclined toward each other while extending outward.

In order to facilitate the further sewing of the third flap with the stent and ensure the shielding effect, in one embodiment, the width of the third flap 5 is greater than the width of the second flap 4. The third flap 5 does not extend with the same width in the direction of the center line L0, so the width of the third flap 5 refers to the dimension of the widest portion of the third flap 5.

Referring to FIG. 3, depending on the extension direction of the flaps and taking the width direction W0 of the leaflet body 1 as a reference, the included angle between the first fold line L1 and the width direction W0 toward the center line L0 is the first inclination angle β1, the included angle between the second fold line L2 and the width direction W0 toward the center line L0 is the second inclination angle β2, and the included angle between the third fold line L3 and the width direction W0 toward the center line L0 is the third inclination angle β3. The first, second and third inclination angles β1, β2, β3 are all not greater than 90 degrees, for example, 90 degrees.

In the above embodiments, the span of the fixed edge 11 is larger than that of the movable edge 12 in the width direction of the leaflet body 1. Referring to FIG. 3, in the case where the leaflet body 1 has a symmetrical structure (the axis of symmetry is the center line L0), the span of the fixed edge 11 refers to the distance between the end points B of the fixed edge on two sides of the center line L0, and similarly, the span of the movable edge 12 refers to the distance between the end points A of the movable edge on two sides of the center line L0.

Referring to FIGS. 3 to 7, processing the leaflet for the heart valve according to the above embodiments in use includes: the first flap 3 is turned over outward along the first fold line L1 and is overlapped with the pulling portion 2, as shown in FIG. 4, the corner 33 can be sewn to the pulling portion 2 in this step or in the following step.

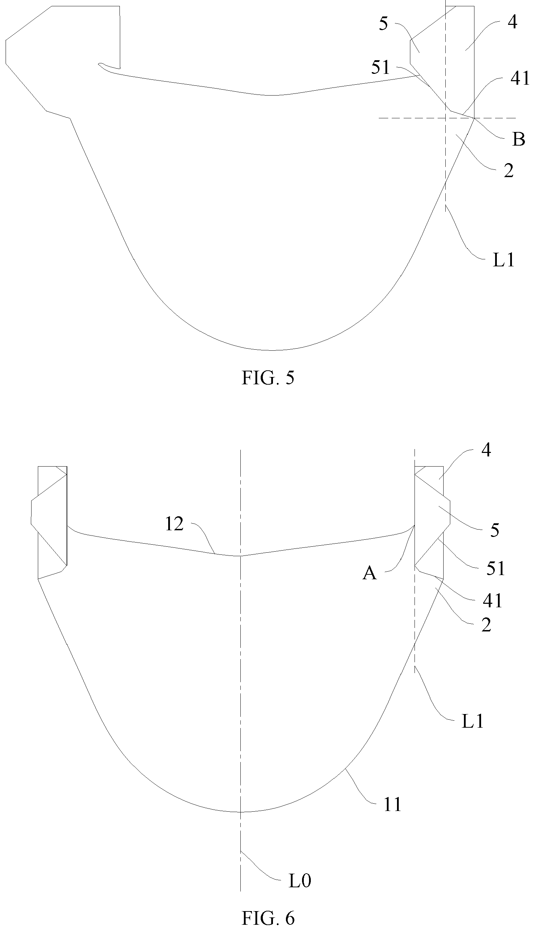

Referring to FIG. 5, the second flap 4 is turned over inward along the second fold line L2 and is overlapped with and covers the first flap 3. In this case, the first fold line L1 is substantially aligned with the third fold line L3, and depending on the inclined tendency of the bottom edge 41 of the second flap 4, the bottom edge 41 of the second flap 4 is inclined upward toward the center line L0 with respect to the horizontal reference line (horizontal dotted line) shown in the figure. The inclination angle ranges from 15 to 45 degrees so that blind angle and blood pack can be avoided as much as possible, thereby reducing the risk of thrombus. The third flap 5 is not configured to be directly sewn to the second flap 4, and it is not strictly limited in this step to whether or not to turn over the third flap 5.

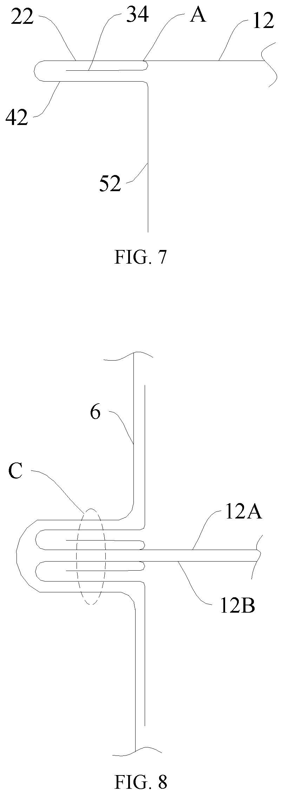

As can be seen in FIG. 7, after the folding of the connecting ear is completed, the overlapping relationship can be better understood in view of the relationship of the top edges of the portions of the connecting ear, wherein the top edge 34 of the first flap is located on one side of the top edge 22 of the pulling portion, the top edge 42 of the second panel is located on the same side, with the top edge 34 of the first flap sandwiched. It is not strictly limited in this step to whether or not to turn over the top edge 52 of the third flap.

The fold lines in the embodiments of the present application can be used as boundaries for the different portions of the connecting ear, and also as references for turning over the portions during processing. In order to simply turn over the portions, the portions for the fold lines can be treated with indentation. A strip of indentation or a plurality of indentations at intervals can be provided.

Alternatively, the portions for the fold lines can be partially thinned, for example, by grinding, chemical treatment, or changing the texture of the weave by omitting the warps or wefts.

In the case where the sealing effect has already been ensured or the sealing requirement is not high, the portions for the fold lines can be pre-perforated, and the small holes are arranged at intervals along the respective fold line.

Referring to FIG. 7 and FIG. 8, an embodiment according to the present application further provides a heart valve preform which includes at least two leaflets and an inner covering film. The specific structure of the leaflet can refer to the above embodiments. The pulling portion and the first flap of the same leaflet are overlapped with each other to form a reinforced portion, and the reinforced portions of two adjacent leaflets are attached to each other to form an assembly of the reinforced portions. The inner covering film 6 is partially folded to wrap the assembly of the reinforced portions and the wrapping portions are fixed by threading suture, with the fixed edges of the leaflets fixed in the inner covering film 6.

The heart valve preform in this embodiment can be stored separately after being prepared, or can be directly subjected to the next step, that is, being sewn to the stent. In practice, it is also possible to combine the step of preparing the heart valve preform with the step of sewing the same to the stent.

Referring to FIGS. 8 to 11, an embodiment of the present invention further provides a heart valve, which includes a stent 7 having a blood flow passage defined therein. Three leaflets for controlling the opening and closing of the blood flow passage are arranged in the blood flow passage. The specific structure of the leaflets can refer to the embodiments described above.

The movable edges 12 of the leaflets cooperate with each other and have a closed state of being engaged with each other and an open state of being separated from each other.

Specifically, the fixed edges 11 of the leaflets can be directly sewn to the stent 7. Alternatively, an inner covering film 6 can be arranged on the inner wall of the stent 7, and the fixed edges 11 and the pulling portions of the leaflets can be directly sewn to the inner covering film 6. As the inner covering film 6 is sewn to the stent 7, the fixed edges 11 of the leaflets are also indirectly sewn to the stent 7.

Alternatively, an outer covering film (not shown in the FIG) can be arranged on the outer wall of the stent 7, and the fixed edges 11of the leaflets are directly sewn to the outer covering film. As the outer covering film is sewn to the stent 7, the fixed edges 11 of the leaflets are also indirectly sewn to the stent 7.

Alternatively, the inner covering film 6 can be combined with the outer covering film, and the fixed edges 11 of the leaflets, depending on the respective locations, are directly sewn to at least one of the stent 7, the inner covering film 6 and the outer covering film.

Taking the inner covering film 6 as an example and referring to FIGS. 8 to 11, an embodiment of the present application further provides a heart valve including a stent 7, in which a blood flow passage is defined, the inner covering film 6 is disposed on the inner wall of the stent 7, and three flaps for controlling the opening and closing of the blood flow passage are sewn on the inner covering film 6. The specific structure of the leaflets can refer to the embodiments described above.

The heart valve can be applied to various locations of the heart and the peripheries thereof. For example, the heart valve can be used as a pulmonary valve, i.e., be applied at the pulmonary artery.

The fixed edges 11 and the pulling portions 2 of the leaflets are all connected with the inner covering film 6, and the movable edges 12 of the leaflets cooperate with each other and have a closed state of being engaged with each other and an open state of being separated from each other.

The material and specific structure of the stent 7 and the inner covering film 6 can refer to the prior art, which are not the focus of the present application. The inner covering film 6 is fixed on the inner wall of the stent through a first suture. The pulling portion 2 and the first flap 3 are overlapped with each other to form a reinforced portion, the reinforced portions of two adjacent leaflets are attached to each other to form an assembly of the reinforced portions. The inner covering film 6 is partially folded to wrap the assembly of the reinforced portions, and the wrapping portions are fixed by a second suture.

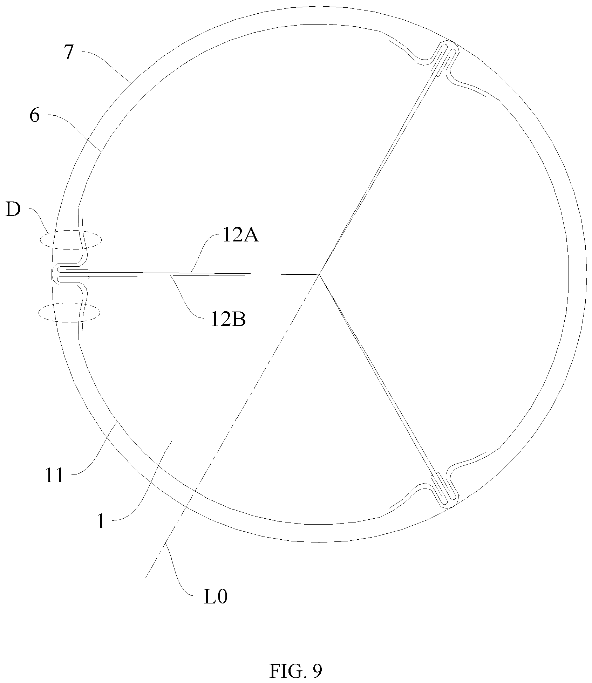

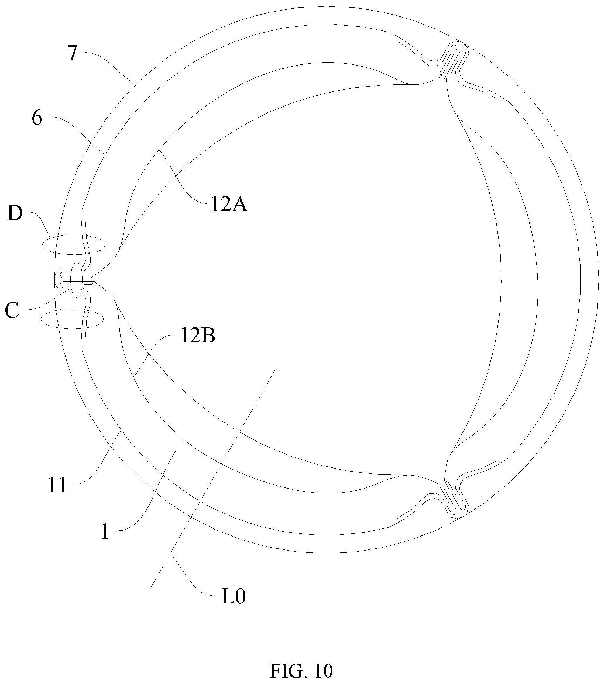

Referring to FIGS. 9 and 10, in the closed and open state of the blood flow passage inside the stent 7, the movable edges 12A and 12B of the two adjacent leaflets are closed to each other or opened separately.

After the inner covering film 6 is partially folded thereby wrapping the assembly of the reinforced portions, it is fixed with the assembly of the reinforced portions by the second suture at the sewing area C. In other embodiments, the outer side of the pulling portion 2 is further connected with a second flap 4. The reinforced portion further includes the second flap 4 overlapping with the pulling portion 2.

In other embodiments, the outer side of the second flap 4 is further connected with a third flap 5 which extends in the circumferential direction of the stent and contacts the inner covering film 6 and is connected to the stent 7 by a third suture at the contact portion. As it can be seen from FIG. 10, after the third flap 5 contacts the inner covering film 6, the third flap 5 is sewn to the stent 7 by means of a third suture at the sewing area D. The figure is only shown for easy illustration of the connection, in practice, under the pulling of the third flap 5, the assembly of the reinforced portion will close to the inner covering film 6 and the inner wall of the stent 7 as much as possible.

The stent 7 can be formed by tube cutting or by weaving. Depending on the application, the stent 7 can be a surgical biovalve stent or an interventional biovalve stent in the prior art.

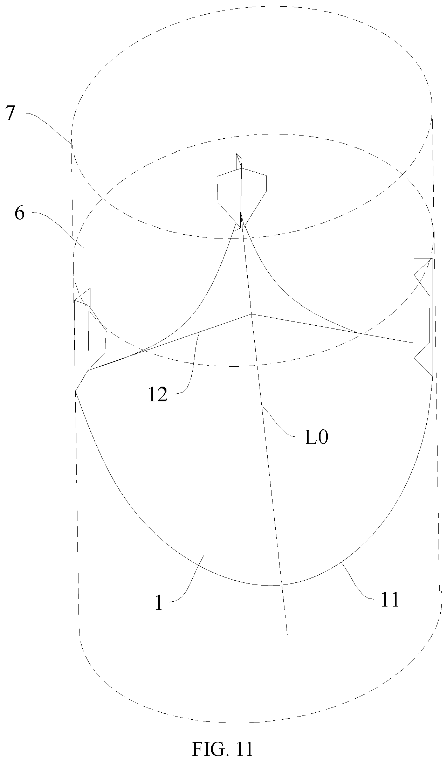

In a preferred embodiment, at least a section of the stent 7 in the axial direction is a straight cylindrical section. As shown in FIG. 11, only the straight cylindrical section of the stent is illustrated. The pulling portion 2 is axially located on the straight cylindrical section, and the extension direction of the pulling portion 2 is parallel to the axial direction of the straight cylindrical section.

After the leaflet for the heart valve is sewn to the stent 7, it is no longer of a plane structure. The extension direction of the pulling portion 2 is no longer parallel to the center line L0 of the leaflet body 1. The pulling portion 2 is configured to be sewn to the stent 7 and adapted to the structure of the stent 7, and the extension direction of the pulling portion 2 is parallel to the axial direction of the straight cylindrical section. In other words, the pulling portion 2 extends along the generatrix of the straight cylindrical section, so that the pulling portion 2 can better pull the leaflet body 1 in the axial direction of the straight cylindrical section, thereby keeping the movable edges 12A and 12B flat and close to each other in the closed state even when subjected to the impact of the backflow blood. Furthermore, due to the stress release opening, there is good stress response between the movable edge and the reinforced portion.

In order to connect the third flap 5 to the stent 7 better, in a preferred embodiment, the stent 7 has a structure of cells, with each cell including edge strips and a hollowed-out area surrounded by the edge strips. The third flaps 5 of two adjacent leaflets are configured to be fixed to the edge strips of one of the cells by a third suture.

For example, the cell can be rhomboid, and the third flaps 5 of the two adjacent leaflets are configured to be engaged with each other and fixed to the respective edge strips of the rhomboid cell, which further improves the strength and prevents the edges of the third flap 5 from floating under the influence of blood flow.

An embodiment according to the present application further provides a method for preparing the pulmonary valve, which includes connecting the leaflets to the inner covering film to form a preform, and then fixing the preform to a stent.

Referring to FIGS. 3 to 10, in preparing the preform, the pulling portion 2 and the first flap 3 of the same leaflet are overlapped with each other to form a reinforced portion, and the reinforced portions of two adjacent leaflets are attached to each other to form an assembly of the reinforced portions; the inner covering film 6 is partially folded to wrap the assembly of the reinforced portions, and the wrapping portions are fixed by threading a second suture; and the fixed edge 11 is fixed to the inner covering film 6.

When the fixed edge 11 is fixed to the inner covering film 6, the fixed edge 11 can be further fixed to the stent 7, which can be considered as one of the steps of fixing the preform to the stent 7. Fixing the preform to the stent can include only sewing the fixed edge 11, sewing the third flap 5, and sewing the other portions of the inner covering film 6, the order for the three steps is not strictly limited and can be adjusted based on the operating habits.

In the present application, by improving the shape of the connecting ear and the positional relationship between the connecting ear and the leaflet body, the force applied to the movable edge is reasonably distributed, and the folded portions are as flat as possible thereby eliminating unnecessary stress and reducing the risk of thrombus.

The features described in the above various embodiments can be combined. In order to simplify the descriptions, not all possible combinations of the features in the above embodiments have been described. However, any combinations of the features should be within the scope of the invention as long as no conflict resides among these features. In the case where the features in different embodiments are shown in the same drawing, it may be considered that this drawing discloses a combination of the various embodiments involved.

The above embodiments are only several implementations of the present invention which are described specifically and in detail, without limitation to the scope claimed by the present invention. Those skilled in the art can make various modifications and variations to the embodiments without departing from the spirit and scope of the present invention, and these modifications and variations should fall into the scope claimed by the present invention. Therefore, the scope of protection of the invention patent should be subject to the attached claims.

## Claims

1. An improved leaflet for a heart valve, comprising a leaflet body which, in a height direction, has a movable edge on a top side and a fixed edge on a bottom side, the movable edge and the fixed edge extending in a width direction of the leaflet body and connected to connecting ears on respective sides, wherein the connecting ear comprises:
a pulling portion extending in the height direction and crossing over the movable edge, the movable edge extending to and meeting with an inner side of the pulling portion, and the fixed edge extending to and meeting with an outer side of the pulling portion; and
a first flap connected to the inner side of the pulling portion, with a first fold line defined between the first flap and the inner side of the pulling portion, wherein one side of the first flap facing the movable edge is a bottom edge, and a stress release opening is defined between the bottom edge and the movable edge to allow the first flap to be turned over along the first fold line.

2. The improved leaflet for the heart valve according to claim 1, wherein a middle portion of the fixed edge protrudes toward the bottom side of the leaflet body in a parabolic shape; and
the movable edge extends straightly, or a middle portion of the movable edge also protrudes toward the bottom side of the leaflet body.

3. The improved leaflet for the heart valve according to claim 2, wherein a line connecting a midpoint of the movable edge and a midpoint of the fixed edge is a center line of the leaflet body, and the connecting ears on two sides of the leaflet body are symmetrically or asymmetrically distributed on two sides of the center line.

4. The improved leaflet for the heart valve according to claim 1, wherein the pulling portion is a strip, and in a length direction of the strip, one side of the pulling portion is a bottom edge which is a part of the fixed edge, and the other side of the pulling portion is a top edge, and wherein a top edge of the first flap and the top edge of the pulling portion are flush with or offset from each other.

5. The improved leaflet for the heart valve according to claim 4, wherein portions of the top edges of the first flap and the pulling portion adjacent to the first fold line are symmetrical about the first fold line.

6. The improved leaflet for the heart valve according to claim 4, wherein one end of the top edge of the first flap is flush with the top edge of the pulling portion, and the other end extends in the width direction of the leaflet body or is inclined toward the bottom edge of the first flap.

7. The improved leaflet for the heart valve according to claim 4, wherein the pulling portion extends with the same width after crossing over an end of the movable edge.

8. The improved leaflet for the heart valve according to claim 4, wherein the first flap is a strip and extends with the same width after crossing over an end of the movable edge.

9. The improved leaflet for the heart valve according to claim 4, wherein one end of the bottom edge of the first flap meets the inner side of the pulling portion, and the other end extends in the width direction of the leaflet body or obliquely with respect to the width direction.

10. The improved leaflet for the heart valve according to claim 4, wherein a width of the first flap is less than a width of the pulling portion and greater than 50% of the width of the pulling portion.

11. The improved leaflet for the heart valve according to claim 4, wherein the first flap is configured to be overlapped with the pulling portion in use.

12. The improved leaflet for the heart valve according to claim 4, wherein the first flap has a corner at a bottom thereof and at a side thereof away from the pulling portion, and in the height direction of the leaflet body, the corner is located below an end of the movable edge.

13. The improved leaflet for the heart valve according to claim 1, wherein the stress release opening is strip-shaped and extends with the same width or is narrowed gradually.

14. The improved leaflet for the heart valve according to claim 1, wherein the stress release opening is strip-shaped and extends along a straight line or a curved line.

15. The improved leaflet for the heart valve according to claim 13, wherein the stress release opening has a closed end adjacent to the first fold line, and the closed end has an arcuate inner edge.

16. The improved leaflet for the heart valve according to claim 13, wherein the stress release opening extends along a curved line and curves gradually toward a top edge of the first flap as the stress release opening approaches the first fold line.

17. The improved leaflet for the heart valve according to any one of claims 1 to 16, wherein the outer side of the pulling portion is further connected with a second flap, a bottom edge of the second flap is connected with an end of the fixed edge, and the connection portion is a turning point, and wherein a second fold line extends from the turning point upward between the pulling portion and the second flap.

18. The improved leaflet for the heart valve according to claim 17, wherein the second flap is a strip, and a middle portion of the second flap in a length direction thereof extends with the same width in the height direction of the leaflet body.

19. The improved leaflet for the heart valve according to claim 17, wherein a width of the second flap is less than a width of the pulling portion and greater than 50% of the width of the pulling portion.

20. The improved leaflet for the heart valve according to claim 19, wherein the second flap is configured to be overlapped with the pulling portion in use.

21. The improved leaflet for the heart valve according to claim 20, wherein the second flap and the first flap are configured to be respectively overlapped on two sides of the pulling portion or on the same side of the pulling portion in use.

22. The improved leaflet for the heart valve according to claim 20, wherein the second flap and the first flap are configured to be respectively overlapped on the same side of the pulling portion in use, with the first flap sandwiched between the second flap and the pulling portion.

23. The improved leaflet for the heart valve according to claim 17, wherein an angle at the turning point is an obtuse angle.

24. The improved leaflet for the heart valve according to claim 17, wherein one end of a top edge of the second flap is flush with a top edge of the pulling portion, and the other end extends in the width direction of the leaflet body or is inclined toward the bottom edge of the second flap.

25. The improved leaflet for the heart valve according to claim 17, wherein one end of the bottom edge of the second flap is flush with a bottom edge of the pulling portion, and the other end extends in the width direction of the leaflet body or is inclined toward a top edge of the second leaflet.

26. The improved leaflet for the heart valve according to claim 17, wherein an outer side of the second flap is further connected with a third flap with a third fold line defined between the second flap and the third flap, and the third flap is configured to be directly or indirectly attached to a support in use.

27. The improved leaflet for the heart valve according to claim 26, wherein top and bottom edges of the third flap are gradually inclined toward each other while extending outward.

28. The improved leaflet for the heart valve according to claim 27, wherein a width of the third flap is greater than a width of the second flap.

29. The improved leaflet for the heart valve according to claim 1, wherein the fixed edge has a larger span than the movable edge in the width direction of the leaflet body.

30. A heart valve preform, comprising at least two leaflets and an inner covering film, wherein the leaflet is the improved leaflet for the heart valve according to any one of claims 1 to 29, and wherein the pulling portion and the first flap of the same leaflet are overlapped with each other to form a reinforced portion, and the reinforced portions of two adjacent leaflets are attached to each other to form an assembly of the reinforced portions; and the inner covering film is partially folded to wrap the assembly of the reinforced portions, and the wrapping portions are fixed by threading suture; and the fixed edges of the leaflets are fixed with the inner covering film.

31. A heart valve, comprising a stent having a blood flow passage therein, in which three leaflets for controlling opening and closing of the blood flow passage are arranged, and wherein the leaflet is the improved leaflet for the heart valve according to any one of claims 1 to 29, and the movable edges of the leaflets cooperate with each other and have a closed state of being engaged with each other and an open state of being separated from each other.

32. The heart valve according to claim 31, wherein an inner covering film is provided on an inner wall of the stent, and the fixed edges and the pulling portions of the leaflets are all connected with the inner covering film.

33. The heart valve according to claim 32, wherein the inner covering film is fixed with the inner wall of the stent by a first suture, and the pulling portion and the first flap are overlapped to form a reinforced portion, and the reinforced portions of two adjacent leaflets are attached to each other to form an assembly of the reinforced portions; and the inner covering film is partially folded to wrap the assembly of the reinforced portions, and the wrapping portions are fixed by a second suture.

34. The heart valve according to claim 33, wherein an outer side of the pulling portion is further connected with a second flap, and the reinforced portion further comprises the second flap overlapping with the pulling portion.

35. The heart valve according to claim 34, wherein the outer side of the second flap is further connected with a third flap extending in a circumferential direction of the stent and contacting the inner covering film, with the contact portion connected with the stent by a third suture.

36. The heart valve according to claim 35, wherein the stent has a structure of cells with each cell having edge strips and a hollowed-out area surrounded by the edge strips, and the third flaps of two adjacent leaflets are fixed with the edge strips of one of the cells by the third suture.

37. The heart valve according to claim 32, wherein at least a section of the stent in an axial direction is a straight cylindrical section, and the pulling portion is axially located on the straight cylindrical section, and an extension direction of the pulling portion is parallel to an axial direction of the straight cylindrical section.

38. The improved heart valve according to claim 31, wherein the heart valve is a pulmonary valve.

39. A method for preparing a heart valve, comprising connecting leaflets for the heart valve with an inner covering film to prepare a preform, and then fixing the preform to a stent, wherein the leaflet is the improved leaflet for the heart valve according to any one of claims 1 to 29, and preparing the preform comprises:
overlapping the pulling portion with the first flap of the same leaflet to form a reinforced portion;
attaching the reinforced portions of two adjacent leaflets together to form an assembly of the reinforced portions;
folding the inner covering film partially to wrap the assembly of the reinforced portions, and threading suture through and thus fixing the wrapping portions; and
fixing the fixed edges of the leaflets to the inner covering film.
